# EUROPÄISCHE PATENTANMELDUNG

(11) **EP 2 266 508 A1**
(43) Veröffentlichungstag der Anmeldung: **29.12.2010**
(21) Anmeldenummer: 10158587.5
(22) Anmeldetag: 31.03.2010
(51) Int. Cl.: A61F 2/90, A61F 2/02, A61F 2/00

(54) **Stent mit verbessertem Stentdesign**

(30) Priorität: 25.06.2009 US 220212 P
(71) Anmelder: Biotronik VI Patent AG, 6341 Baar (CH)
(72) Erfinder: Götzen, Nils, 18057, Rostock (DE); Schettler, Jan, 18053, Rostock (DE)
(74) Vertreter: Lindner-Vogt, Karin L.

(57) **Zusammenfassung**

Expandierbaren Stent aufweisend eine Mehrzahl von umlaufenden Stützstrukturen (1,2), die nacheinander folgend entlang der longitudinalen Achse (5) angeordnet sind und jeweils aus einer Aneinanderreihung von Diagonalelementen (4a,4b) und Bogenelementen (6) aufgebaut sind; und einen oder mehrere Konnektoren (3), wobei zwei aufeinander folgende umlaufende Stützstrukturen über mindestens einen Konnektor miteinander verbunden sind und wobei ein Konnektor an jeweils ein Diagonalelement der beiden zu verbindenden umlaufenden Stützstrukturen angebunden ist; wobei ein Diagonalelement, an welches ein Konnektor angebunden ist, eine langgestreckte Form mit zwei entgegengesetzten Enden aufweist; an seinem ersten Ende eine Verzweigungsstelle mit einem Durchmesser d₁ aufweist, an der das Diagonalelement sich unmittelbar in ein Bogenelement und einen Konnektor verzweigt; an seinem zweiten Ende eine Verbindungsstelle mit einem Durchmesser d₂ aufweist, an der das Diagonalelement unmittelbar in ein weiteres Bogenelement übergeht; und wobei das Verhältnis von d₁ zu d₂ > 1 ist und sich das Diagonalelement von seinem ersten Ende zu seinem zweiten Ende kontinuierlich verjüngt.

## Beschreibung

### Stent mit verbessertem Stentdesign

Die Erfindung betrifft einen Stent mit einem verbesserten Stentdesign.

### Hintergrund der Erfindung

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu einen Grundkörper auf, der eine Vielzahl von umlaufenden Stützstrukturen z.B. aus metallischen Streben aufweist, der zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen einen rohrförmigen Grundkörper von ausreichender Tragkraft, um das verengte Gefäß im gewünschten Maße offen zu halten, durch den der Blutfluss ungehindert weiterläuft. Die Umfangswandung des Grundkörpers wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Der Vorgang der Positionierung und Expansion der Stents während der Prozedur und die abschließende Lage des Stents im Gewebe nach Beendigung der Prozedur muss durch den Kardiologen überwacht werden. Dies kann durch Bildgebende Verfahren, wie z.B. durch Röntgenuntersuchungen erfolgen.

Der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Gebrauch mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und biokorrodierbare/resorbierbare Werkstoffe unterteilt werden.

Stents weisen einen röhrenförmigen Grundkörper auf, der ein Lumen entlang einer longitudinalen Achse umfasst. Der Grundkörper weist eine Mehrzahl von umlaufenden Stützstrukturen auf, z.B. umlaufende zylindrische Mäanderringe oder Helices, die nacheinander folgend entlang der longitudinalen Achse angeordnet sind. Diese Stützstrukturen sind jeweils aus einer Aneinanderreihung von Diagonalelementen und Bogenelementen (auch Kronen genannt) aufgebaut und bilden die geometrische Grundeinheit von modernen Stentdesigns. Die Stützstrukturen werden durch Verbindungselemente - sogenannte Konnektoren - in longitudinaler Richtung miteinander verbunden. Einerseits müssen diese Konnektoren so angeordnet sein, dass sie eine ausreichende Biegeflexibilität des Stents gewährleisten, andererseits dürfen sie einen Crimp- und/oder Dilatationsprozess nicht behindern.

In US 5,810,872 wird ein Stentdesign vorgeschlagen bei dem die Konnektoren am Scheitelpunkt der Bogenelemente der Mäanderstruktur angebracht sind und sich in allgemein longitudinaler Richtung erstrecken. Zwar beeinflussen die Konnektoren in diesem Stentdesign die Crimpbarkeit nur minimal, stören aber die homogene plastische Deformation der Bogenelemente und haben damit einen wesentlichen negativen Einfluss auf die mechanischen Eigenschaften des Stents.

In US 6,613,079 wird ein alternatives Stentdesign vorgestellt, bei dem die Konnektoren direkt in der Mitte der Diagonalelemente, zwischen den beiden Verbindungsstellen des Diagonalelements mit den folgenden Bogenelementen, angebracht sind. Zwar wird bei diesem Stentdesign der Kraftfluss in den Bögen nicht gestört, allerdings führt die mittige Anbindung der Konnektoren an die Diagonalelemente zu Behinderungen beim Crimpen.

Gerade bei der Nutzung von Magnesium als degradierbares Stentmaterial, welches über nicht besonders günstige mechanische Werkstoffeigenschaften verfügt, sind minimale Beeinflussungen des Kraftflusses in Kombination mit einer effektive Nutzung des Crimpraumes von besonderer Bedeutung und erfordern somit ein optimales Konnektoranbindungsdesign.

Der vorliegenden Erfindung liegt die Aufgabe zugrunde, ein oder mehrere der zuvor geschilderten Probleme zu lösen oder zumindest zu mindern. Insbesondere soll ein Stentdesign bereitgestellt werden, welches eine lediglich minimale Beeinflussung des Kraftflusses in den Bogenelementen mit einer effektiven Nutzung des zum Crimpen zur Verfügung stehenden Raumes erlaubt.

Die Aufgabe wird gelöst durch Bereitstellung eines expandierbaren Stents umfassend einen röhrenförmigen Grundkörper mit einem Lumen entlang einer longitudinalen Achse, wobei der Grundkörper:
- eine Mehrzahl von umlaufenden Stützstrukturen aufweist, die nacheinander folgend entlang der longitudinalen Achse angeordnet sind und jeweils aus einer Aneinanderreihung von Diagonalelementen und Bogenelementen aufgebaut sind; und
- einen oder mehrere Konnektoren aufweist, wobei zwei aufeinander folgende umlaufende Stützstrukturen über mindestens einen Konnektor miteinander verbunden sind und wobei ein Konnektor an jeweils ein Diagonalelement der beiden zu verbindenden umlaufenden Stützstrukturen angebunden ist;
**dadurch gekennzeichnet, dass**
mindestens ein Diagonalelement, an welches ein Konnektor angebunden ist, eine langgestreckte Form mit zwei entgegengesetzten Enden aufweist,
i) wobei das Diagonalelement an seinem ersten Ende eine Verzweigungsstelle mit einem Durchmesser d₁ aufweist, an der das Diagonalelement sich unmittelbar in ein Bogenelement und einen Konnektor verzweigt, und
ii) wobei das Diagonalelement an seinem zweiten Ende eine Verbindungsstelle mit einem Durchmesser d₂ aufweist, an der das Diagonalelement unmittelbar in ein weiteres Bogenelement übergeht; und
iii) wobei das Verhältnis von d₁ zu d₂ > 1 ist und sich das Diagonalelement von seinem ersten Ende zu seinem zweiten Ende kontinuierlich verjüngt.

Die erfindungsgemäße Lösung zeichnet sich dadurch aus, dass sich der Übergangsbereich vom Konnektor zur Stützstruktur über den gesamten Bereich eines Diagonalelements erstreckt, aber nicht in eine Bogenstruktur hineinreicht. Dadurch, dass sich die Anbindung des Konnektors auf ein Diagonalelement beschränkt und nicht in ein Bogenelement hineinreicht, bleibt eine homogene Verteilung der Spannungen und Dehnungen in den Bogenelementen des Stents unbeeinflusst. Eine homogene plastische Deformierbarkeit der erfindungsgemäßen Stents bleibt so gewährleistet. Dadurch, dass sich die Verbindungsstelle zwischen Konnektor und Diagonalelement an einem näher zur nächsten Stützstruktur liegende Ende des Diagonalelements befindet, beansprucht die Anbindung nur wenig Raum, so dass genügend Raum zur Gewährleistung einer ausreichenden Crimpbarkeit und Biegeflexibilität des erfindungsgemäßen Stents zur Verfügung steht. Dadurch, dass die Verbindung zwischen Konnektor und Diagonalelement über die gesamte Länge des Diagonalelements abgestützt wird, weist der erfindungsgemäße Stent eine optimale Kraftübertragung von einer Stützstruktur zur nächsten, sowie die notwendige Stabilität auf. Eine Ausrichtung der Konnektoren im Wesentlichen entlang der longitudinalen Achse und/oder ein spitzwinkliger Übergang vom Konnektor zum Diagonalelement führen zu einer optimalen Raumausnutzung im gecrimpten Zustand des erfindungsgemäßen Stents.

Der erfindungsgemäße expandierbare Stent weist einen röhrenförmigen Grundkörper auf, der ein Lumen entlang einer longitudinalen Achse umschließt. Durch dieses Lumen kann, nach erfolgter Applikation des Stents in ein Blutgefäß, ein Blutfluss erfolgen. Der Grundkörper umfasst eine Mehrzahl von umlaufenden Stützstrukturen, die nacheinander folgend entlang der longitudinalen Achse angeordnet sind und das Lumen umschließen. Die Stützstrukturen sind jeweils aus einer Aneinanderreihung von Diagonalelementen und Bogenelementen aufgebaut. Die Diagonalelemente weisen eine langgestreckte Form mit zwei Enden auf und verbinden zwei Bogenelemente mit gegenläufig ausgerichteter Krümmung. Die Diagonalelemente sind im Wesentlichen für die Ausdehnung einer Stützstruktur in longitudinaler Achsenrichtung verantwortlich. Die Bogenelemente sind gekrümmt und verbinden zwei aufeinander folgende Diagonalelemente einer Stützstruktur derart miteinander, dass diese entlang einer zur longitudinalen Achse vertikal verlaufenden Achse übereinander zu liegen kommen, wobei eine ringförmige umlaufende Struktur entsteht, die ein Lumen umschließt.

Der Grundkörper umfasst neben einer Mehrzahl von Stützstrukturen einen oder mehrere Konnektoren, wobei zwei aufeinander folgende umlaufende Stützstrukturen über mindestens einen Konnektor miteinander verbunden sind. Die Konnektoren des erfindungsgemäßen Stents sind derart ausgestaltet, dass eine Mehrzahl von Stützstrukturen zu einem Grundkörper verbunden werden kann, der für den Einsatz in einem expandierbaren Stent geeignet ist. Dazu ist jeweils ein Konnektor an einem ersten Ende mit einem Diagonalelement einer ersten Stützstruktur verbunden und an einem zweiten Ende mit einem Diagonalelement einer zweiten Stützstruktur. Zwei aufeinander folgende Stützstrukturen können auch über mehr als einen Konnektor miteinander verbunden sein. Einer, mehrere oder alle Konnektoren des erfindungsgemäßen Stents können eine langgestreckte Form mit zwei entgegengesetzten Enden aufweisen. Bevorzugt sind die Konnektoren nur so lang, dass eine ausreichende Flexibilität der beiden benachbarten Stützstrukturen gewährleistet ist, aber nicht so lang, dass der erfindungsgemäße Stent torsionsweich wird. Einer, mehrere oder alle Konnektoren eines erfindungsgemäßen Stents können eine geschwungene Form aufweisen. Einer, mehrere oder alle Konnektoren eines erfindungsgemäßen Stents können jeweils in einem spitzen Winkel vom Diagonalelement abzweigen. Die Konnektoren sind in grundsätzlich longitudinaler Richtung zwischen den beiden zu verbindenden umlaufenden Stützstrukturen ausgerichtet, wobei die Konnektoren nicht notwendigerweise in paralleler Ausrichtung zur longitudinalen Achse vorliegen. Die Konnektoren können im dilatiertem Zustand des Stents zur longitudinalen Achse einen Winkel □ von ≥ 0° und < 70° bilden, bevorzugt von ≥ 0° und < 45°, besonders bevorzugt von ≥ 0° und < 25°. Unter dilatiertem Zustand kann sowohl der Zustand des Stents vor einem Crimpen des Stents auf eine geeignete Applikationsform verstanden werden, als auch der Zustand des erfindungsgemäßen Stents nach einer Implantation des Stents. Der erfindungsgemäße Stent weist dabei mindestens in einer der beiden Formen des dilatierten Zustands einen Winkel □ aus den oben genannten Bereichen auf.

Der erfindungsgemäße Stent weist Diagonalelemente auf, an welche ein Konnektor angebunden ist. Ein Diagonalelement, an welches ein Konnektor gebunden ist, besitzt eine langgestreckte Form mit zwei entgegengesetzten Enden. Am ersten Ende weist das Diagonalelement eine Verzweigungsstelle auf mit einem Durchmesser d₁. An der Verzweigungsstelle geht das Diagonalelement direkt und unmittelbar in ein Bogenelement und einen Konnektor über. An seinem zweiten Ende weist das Diagonalelement eine Verbindungsstelle mit einem Durchmesser d₂ auf. An der Verbindungsstelle geht das Diagonalelement direkt und unmittelbar in ein weiteres Bogenelement über. Das Verhältnis von d₁ zu d₂ ist > 1 und beträgt bevorzugt zwischen 1,1 und 5, besonders bevorzugt zwischen 1,2 und 3, ganz besonders bevorzugt zwischen 1,25 und 2,5. Das Diagonalelement verjüngt sich von seinem ersten Ende hin zu seinem zweiten Ende. Diese Verjüngung kann gleichmäßig erfolgen. Die Verjüngung kann aber auch ungleichmäßig erfolgen, wobei die Verjüngung in einem dem ersten Ende zugewandten Bereich des Diagonalelements stärker ausgeprägt ist als in einem dem zweiten Ende zugewandten Bereich. Insbesondere ist die Anbindung des Konnektors an das Diagonalelement über die gesamte Länge des Diagonalelements abgestützt und kann organisch ausgebildet sein. Bei einem erfindungsgemäßen Stent können ein, mehrere oder alle Konnektor-Verbindungen zwischen den Stützstrukturen jeweils über oben beschriebene Diagonalelemente erfolgen.

Der Grundkörper des erfindungsgemäßen Stents kann aus jedem Implantatwerkstoff bestehen, der für die Herstellung von Implantaten, insbesondere Stens, geeignet ist. Implantatwerkstoffe für Stents umfassen Polymere, metallische Werkstoffe und keramische Materialien. Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiAl6V4 oder TiAl6Nb7) und Goldlegierungen. Bevorzugt weist der Grundkörper einen metallischen Implantatwerkstoff auf oder besteht daraus.

Besonders bevorzugt weist der erfindungsgemäße Stent einen Grundkörper auf, der einen biodegradierbaren Implantatwerkstoff enthält oder daraus besteht. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen. Insbesondere kann der Grundkörper eines erfindungsgemäßen Stents eine biokorrodierbare Magnesiumlegierung aufweisen oder daraus bestehen.

Unter Legierung wird vorliegend ein metallisches Gefüge verstanden, deren Hauptkomponente Magnesium, Eisen, Zink oder Wolfram ist. Hauptkomponente ist die Legierungskomponente, deren Gewichtsanteil an der Legierung am höchsten ist. Ein Anteil der Hauptkomponente beträgt vorzugsweise mehr 50 Gew.%, insbesondere mehr als 70 Gew.%.

Die Legierungen der Elemente Magnesium, Eisen, Zink oder Wolfram sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Der Begriff Korrosion bezieht sich vorliegend auf die Reaktion eines metallischen Werkstoffes mit seiner Umgebung, wobei eine messbare Veränderung des Werkstoffs bewirkt wird, die - bei Einsatz des Werkstoffs in einem Bauteil - zu einer Beeinträchtigung der Funktion des Bauteils führt. Ein Korrosionssystem besteht vorliegend aus dem korrodierenden metallischen Werkstoff sowie einem flüssigen Korrosionsmedium, das in seiner Zusammensetzung die Verhältnisse in physiologischer Umgebung nachstellt oder ein physiologisches Medium, insbesondere Blut ist. Werkstoffseitig beeinflussen die Korrosion Faktoren, wie die Zusammensetzung und Vorbehandlung der Legierung, mikro- und submikroskopische Inhomogenitäten, Randzoneneigenschaften, Temperatur- und Spannungszustand und insbesondere die Zusammensetzung einer die Oberfläche bedeckenden Schicht. Auf Seiten des Mediums wird der Korrosionsprozess durch Leitfähigkeit, Temperatur, Temperaturgradienten, Azidität, Volumen-Oberflächen-Verhältnis, Konzentrationsunterschied sowie Strömungsgeschwindigkeit beeinflusst.

In DE 197 31 021 A1 werden geeignete biokorrodierbare metallische Implantatwerkstoffe vorgestellt, deren Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest < 1% bekannt, die sich insbesondere zur Herstellung einer Endoprothese, z. B. in Form eines selbstexpandierenden oder ballonexpandierbaren Stent, eignet.

Figuren:
- Figur 1: zeigt schematisch einen Ausschnitt aus einem Grundkörper eines erfin- dungsgemäßen Stents.
- Figur 2: zeigt schematisch einen vergrößerten Ausschnitt aus FIG. 1, wobei die de- taillierte Ausformung eines Diagonalelements gezeigt ist, an welches ein Konnektor angebunden ist.

Die Erfindung wird nachfolgend anhand eines Ausführungsbeispiels näher erläutert.

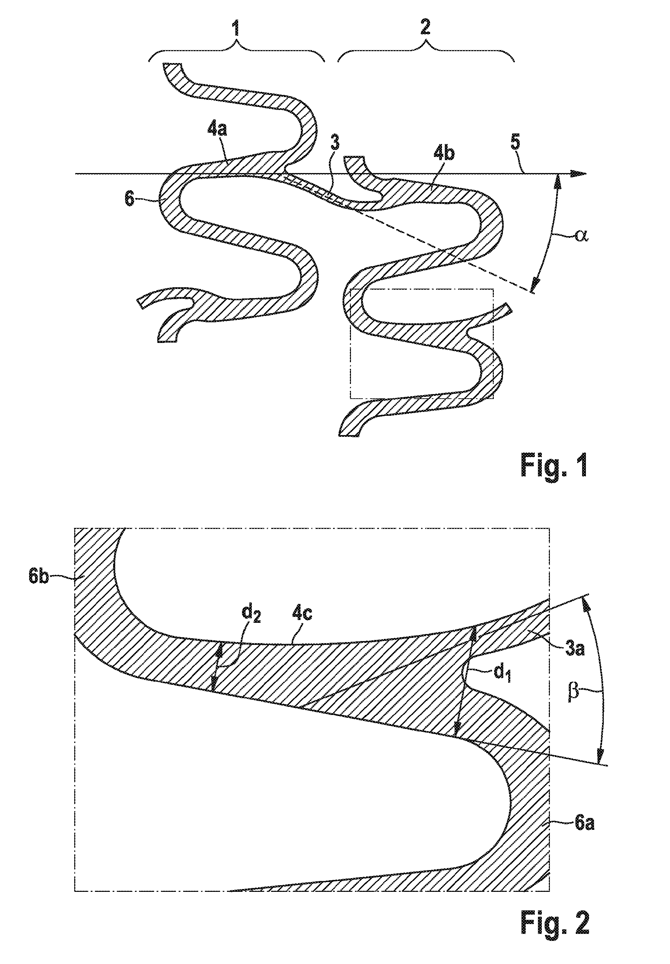

In FIG. 1 ist ein Ausschnitt aus einem Grundkörper eines erfindungsgemäßen Stents gezeigt. Dargestellt sind Abschnitte von zwei aufeinander folgenden Stützstrukturen 1 und 2, die über einen Konnektor 3 miteinander verbunden sind. Die Stützstrukturen 1 und 2 bestehen aus einer Aneinanderreihung von Diagonalelementen (4) und Bogenelementen (6). Der Konnektor 3 verbindet die beiden Stützstrukturen 1 und 2 über die Diagonalelemente 4a und 4b. Dazu ist der Konnektor 3 in longitudinaler Achse ausgerichtet und weist einen Winkel α zur longitudinalen Achse 5 von ≥ 0° und < 70° auf. Der Konnektor 3 weist dabei eine geschwungene Form auf und zweigt von den Diagonalelementen 1 und 2 jeweils in einem spitzen Winkel ab.

In FIG. 2 ist ein Ausschnitt aus FIG. 1 dargestellt, der die detaillierte Ausformung eines Diagonalelements 4c zeigt, an welches der Konnektor 3a angebunden ist. Das Diagonalelement 4c hat eine langgestreckte Form mit zwei entgegengesetzten Enden. An seinem ersten Ende weist das Diagonalelement 4c eine Verzweigungsstelle auf, an der sich das Diagonalelement direkt und unmittelbar in den Konnektor 3a und das Bogenelement 6a verzweigt. An dieser Verzweigungsstelle weist das Diagonalelement 4c einen Durchmesser d₁ auf. An seinem zweiten Ende besitzt das Diagonalelement 4c eine Verbindungsstelle, an der das Diagonalelement 4c direkt und unmittelbar in das Bogenelement 6b übergeht. An dieser Verbindungsstelle weist das Diagonalelement 4c einen Durchmesser d₂ auf. Dabei ist das Diagonalelement 4c derart ausgestaltet, dass das Verhältnis von d₁ zu d₂ > 1, insbesondere zwischen 1,25 und 2,5, ist. Das Diagonalelement verjüngt sich kontinuierlich von seinem ersten Ende hin zu seinem zweiten Ende. Dabei erfolgt diese Verjüngung nicht gleichmäßig, sondern ist in einem dem ersten Ende des Diagonalelements 4c zugewandten Bereich stärker ausgeprägt, als in einem dem zweiten Ende des Diagonalelements zugewandten Bereich. Der Anschluss des Konnektors 3a stützt sich somit über die gesamte Länge des Diagonalbereichs 4c ab. Der Konnektor 3a zweigt vom Diagonalelement 4c in einem Winkel β ab, der spitz ist (β = > 0° und < 90°).

Durch die organische Anbindung der Konnektoren an die Diagonalelemente wird die Dehnungs- und Spannungsverteilung in den stark belasteten Bogenelementen des erfindungsgemäßen Stents nicht beeinflusst. Dadurch wird eine optimale Materialnutzung gewährleistet. Zusätzlich wird durch die großflächige Konnektoranbindung eine optimale Kraftübertragung von einer Stützstruktur zur anderen unterstützt. Die axiale Ausrichtung der Konnektoren und der spitzwinklige Anschluss führen weiterhin zu einer optimalen Platzausnutzung im gecrimpten Zustand.

### Bezugszeichenliste:

- 1: erste Stützstruktur
- 2: zweite Stützstruktur
- 3, 3a: Konnektor
- 4a, 4b, 4c: Diagonalelement, an welches ein Konnektor angebunden ist
- 5: longitudinale Achse
- 6, 6a. 6b: Bogenelement
- α: Winkel, zwischen longitudinaler Achse und Konnektor
- β: spitzer Winkel, in dem Konnektor von Diagonalelement abzweigt
- d₁: Durchmesser am ersten Ende des Diagonalelements
- d₂: Durchmesser am zweiten Ende des Diagonalelements

## Patentansprüche

1. Expandierbarer Stent umfassend einen röhrenförmigen Grundkörper mit einem Lumen entlang einer longitudinalen Achse, wobei der Grundkörper:
- eine Mehrzahl von umlaufenden Stützstrukturen (1, 2) aufweist, die nacheinander folgend entlang der longitudinalen Achse (5) angeordnet sind und jeweils aus einer Aneinanderreihung von Diagonalelementen (4a, 4b, 4c) und Bogenelementen (6, 6a, 6b) aufgebaut sind; und
- einen oder mehrere Konnektoren (3, 3a) aufweist, wobei zwei aufeinander folgende umlaufende Stützstrukturen (1, 2) über mindestens einen Konnektor (3, 3a) miteinander verbunden sind und wobei ein Konnektor (3, 3a) an jeweils ein Diagonalelement (4a, 4b, 4c) der beiden zu verbindenden umlaufenden Stützstrukturen (1, 2) angebunden ist;
**dadurch gekennzeichnet, dass**
mindestens ein Diagonalelement (4c) , an welches ein Konnektor (3a) angebunden ist, eine langgestreckte Form mit zwei entgegengesetzte Enden aufweist,
i) wobei das Diagonalelement (4c) an seinem ersten Ende eine Verzweigungsstelle mit einem Durchmesser d₁ aufweist, an der das Diagonalelement (4c) sich unmittelbar in ein Bogenelement (6a) und einen Konnektor (3a) verzweigt,
ii) wobei das Diagonalelement (4c) an seinem zweiten Ende eine Verbindungsstelle mit einem Durchmesser d₂ aufweist, an der das Diagonalelement (4c) unmittelbar in ein weiteres Bogenelement (6b) übergeht; und
iii) wobei das Verhältnis von d₁ zu d₂ > 1 ist und sich das Diagonalelement (4c) von seinem ersten Ende zu seinem zweiten Ende verjüngt.

2. Stent nach Anspruch 1, **dadurch gekennzeichnet, dass** der Konnektor (3, 3a) in einem spitzen Winkel (β) vom Diagonalelement (4a, 4b, 4c) abzweigt.

3. Stent nach einem der vorhergehenden Ansprüche, **dadurch gekennzeichnet, dass** der Konnektor (3, 3a) eine geschwungene Form aufweist.

4. Stent nach einem der vorhergehenden Ansprüche, **dadurch gekennzeichnet, dass** der Konnektor (3, 3a) zwischen den beiden zu verbindenden umlaufenden Stützstrukturen (1, 2) in longitudinaler Richtung ausgerichtet ist, wobei der Konnektor (3, 3a) im dilatierten Zustand des Stents zur longitudinalen Achse (5) einen Winkel (α) von ≥ 0° und < 70° bildet, bevorzugt von ≥ 0° und < 45°, besonders bevorzugt von ≥ 0° und < 25°.

5. Stent nach einem der vorhergehenden Ansprüche, **dadurch gekennzeichnet, dass** das Verhältnis von d₁ zu d₂ zwischen 1,1 und 5 beträgt, bevorzugt zwischen 1,2 und 3, besonders bevorzugt zwischen 1,25 und 2,5.

6. Stent nach einem der vorhergehenden Ansprüche, **dadurch gekennzeichnet, dass** sich das Diagonalelement (4c), an welches ein Konnektor (3a) angebunden ist, von seinem ersten Ende zu seinem zweiten Ende hin gleichmäßig verjüngt.

7. Stent nach einem der Ansprüche 1 bis 5, **dadurch gekennzeichnet, dass** sich das Diagonalelement (4c), an welches ein Konnektor (3a) angebunden ist, von seinem ersten Ende zu seinem zweiten Ende hin ungleichmäßig verjüngt, wobei die Verjüngung in einem dem ersten Ende des Diagonalelements (4c) zugewandten Bereich stärker ist, als in einem dem zweiten Ende zugewandten Bereich.

8. Stent nach einem der vorhergehenden Ansprüche, **dadurch gekennzeichnet, dass** der Grundkörper einen metallischen Implantatwerkstoff aufweist oder daraus besteht.

9. Stent nach einem der vorhergehenden Ansprüche, **dadurch gekennzeichnet, dass** der Grundkörper einen biokorrodierbaren Implantatwerkstoff aufweist oder daraus besteht.

10. Stent nach einem der vorhergehenden Ansprüche, **dadurch gekennzeichnet, dass** der Grundkörper eine biokorrodierbare Magnesiumlegierung aufweist oder daraus besteht.
